(19) Europäisches Patentamt
European Patent Office
Office européen des brevets

(11) Veröffentlichungsnummer: **0 529 468 A1**

# (12) EUROPÄISCHE PATENTANMELDUNG

(21) Anmeldenummer: **92114032.3**

(51) Int. Cl.[5]: **C07D 401/04**, A01N 43/66

(22) Anmeldetag: **18.08.92**

(30) Priorität: **30.08.91 DE 4128789**

(43) Veröffentlichungstag der Anmeldung:
**03.03.93 Patentblatt 93/09**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Heinemann, Ulrich, Dr.**
**Am Sonnenhang 1**
**W-5653 Leichlingen 2(DE)**
Erfinder: **Kuhnt, Dietmar, Dr.**
**Körnerstrasse 5**
**W-5090 Leverkusen(DE)**
Erfinder: **Dehne, Heinz-Wilhelm, Dr.**
**Krischer Strasse 81**
**W-4019 Monheim(DE)**
Erfinder: **Dutzmann, Stefan, Dr.**
**Kosenberg 10**
**W-4010 Hilden(DE)**

## (54) Substituierte Pyridyltriatzine, Verfahren zu ihrer Herstellung, ihre Verwendung und neue Zwischenprodukte.

(57) Neue substituierte Pyridyltriazine der Formel (I)

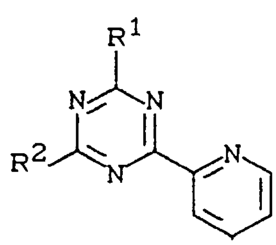

(I)

in welcher $R^1$ und $R^2$ die in der Beschreibung gegebenen Bedeutungen haben und ihre Verwendung zur Bekämpfung von Schädlingen.

Die Verbindungen können nach Analogieverfahren hergestellt werden, z.B. indem man Pyridylamidinhydrochlorid mit geeigneten Imidsäuremethylestern oder mit geeigneten Benzol-isothiocyanaten umsetzt oder indem man mercaptosubstituierte Pyridyltriazine entweder entschwefelt oder mit Alkoholen umsetzt. Die mercaptosubstituierten Verbindungen sind ebenfalls neu und nach Analogieverfahren herstellbar.

EP 0 529 468 A1

Die Erfindung betrifft neue substituierte Pyridyltriazine, mehrere Verfahren zu ihrer Herstellung und ihre Verwendung als Schädlingsbekämpfungsmittel und neue Zwischenprodukte.

Es ist bekannt, daß bestimmte substituierte Pyridylpyrimidine wie beispielsweise die Verbindung 2-(6-Methyl-2-pyridyl)-4-(2-methylphenyl)-pyrimidin oder die Verbindung 2-(6-Methyl-2-pyridyl)-4-phenyl-6-hydroxy-pyrimidin oder die Verbindung 2-(6-Methyl-2-pyridyl)-4-phenyl-6-methoxy-pyrimidin fungizide Eigenschaften besitzen (vgl. z.B. EP 270 362).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Pyridyltriazine der allgemeinen Formel (I),

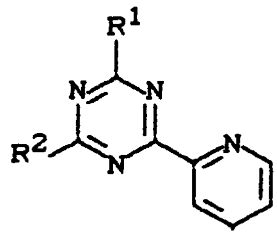

(I)

in welcher

$R^1$ für Wasserstoff, Alkyl, Alkoxy oder Alkylthio steht und

$R^2$ für gegebenenfalls substituiertes Phenyl steht,

gefunden.

Weiterhin wurde gefunden, daß man die neuen substituierten Pyridyltriazine der allgemeinen Formel (I),

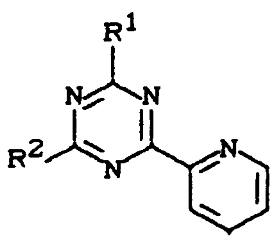

(I)

in welcher

$R^1$ für Wasserstoff, Alkyl, Alkoxy oder Alkylthio steht und

$R^2$ für gegebenenfalls substituiertes Phenyl steht,

erhält, wenn man

a) für den Fall, daß $R^1$ für Alkyl steht, Pyridylamidin oder dessen Säureadditionssalze der Formel (II)

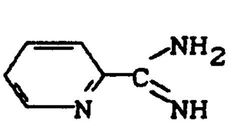 x HX (II)

in welcher

X für ein Anion einer anorganischen Säure steht,

mit Imidsäureestern der Formel (III),

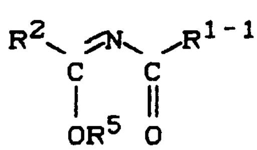

(III)

in welcher

$R^{1-1}$ für Alkyl steht,

$R^2$ die oben angegebene Bedeutung hat, und

$R^5$ für Alkyl oder Aryl steht,

gegebenenfalls in Gegenwart eines Verdünnungsmittels und gegebenenfalls in Gegenwart eines Reaktionshilfsmittels umsetzt oder wenn man

b) für den Fall, daß $R^1$ für Wasserstoff steht, Mercapto-triazinylpyridine der Formel (IV),

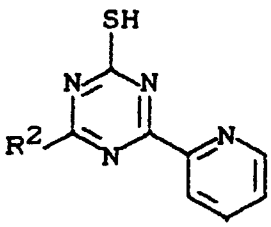

(IV)

in welcher

$R^2$ die oben angegebene Bedeutung hat,

mit Raney Nickel gegebenenfalls in Gegenwart eines Verdünnungsmittels entschwefelt oder

c) für den Fall, daß $R^1$ für Alkylthio steht, die Verbindungen der Formel (IV) mit Alkylierungsmitteln der Formel (V),

$R^3$-E (V)

in welcher

$R^3$ für Alkyl steht und

E für eine elektronenanziehende Abgangsgruppe steht,

gegebenenfalls in Gegenwart eines Verdünnungsmittels und gegebenenfalls in Gegenwart eines Reaktionshilfsmittels umsetzt oder wenn man

d) für den Fall, daß $R^1$ für Alkoxy steht, die mit Hilfe des erfindungsgemäßen Verfahrens (c) erhältlichen Alkylthio-triazinylpyridine der Formel (Ia),

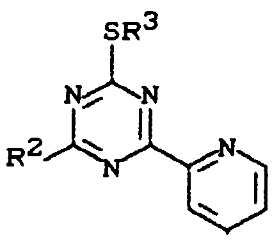

(Ia)

in welcher

$R^3$ für Alkyl steht und

$R^2$ die oben angegebene Bedeutung hat,

mit Alkoholen der Formel (VI),

$R^4$-OH (VI)

in welcher

$R^4$ für Alkyl steht,

gegebenenfalls in Gegenwart eines Verdünnungsmittels und gegebenenfalls in Gegenwart eines Reaktionshilfsmittels umsetzt.

Die Verbindungen der Formel (IV) können auch in der tautomeren Thioketo-Form vorliegen.

Schließlich wurde gefunden, daß die neuen substituierten Pyridyltriazine der allgemeinen Formel (I) eine gute biologische, vor allem fungizide Wirksamkeit besitzen.

Überraschenderweise zeigen die erfindungsgemäßen substituierten Pyridyltriazine der allgemeinen Formel (I) eine deutlich bessere fungizide Wirksamkeit als die aus dem Stand der Technik bekannten substituierten Pyridylpyrimidine, wie beispielsweise die Verbindung 2-(6-Methyl-2-pyridyl)-4-(2-methylphenyl)-pyrimidin oder die Verbindung 2-(6-Methyl-2-pyridyl)-4-phenyl-6-hydroxy-pyrimidin oder die Verbindung 2-(6-Methyl-2-pyridyl)-4-phenyl-6-methoxy-pyrimidin, welches chemisch und wirkungsmäßig naheliegende Verbindungen sind.

Die erfindungsgemäßen substituierten Pyridyltriazine sind durch die Formel (I) allgemein definiert. Bevorzugt sind Verbindungen der Formel (I), bei welchen

$R^1$ für Wasserstoff, für geradkettiges oder verzweigtes Alkyl mit 1 bis 6 Kohlenstoffatomen, für geradkettiges oder verzweigtes Alkoxy mit 1 bis 6 Kohlenstoffatomen oder für geradkettiges oder verzweigtes Alkylthio mit 1 bis 6 Kohlenstoffatomen steht und

$R^2$ für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen oder gegebenenfalls einfach oder mehrfach, gleich oder verschieden durch Halogen und/oder geradkettiges oder verzweigtes Alkyl mit 1 bis 4 Kohlenstoffatomen substituiertes Phenyl.

Besonders bevorzugt sind Verbindungen der Formel (I), bei welchen

$R^1$ für Wasserstoff, für geradkettiges oder verzweigtes Alkyl mit 1 bis 4 Kohlenstoffatomen, für geradkettiges oder verzweigtes Alkoxy mit 1 bis 4 Kohlenstoffatomen oder für geradkettiges oder verzweigtes Alkylthio mit 1 bis 4 Kohlenstoffatomen steht und

$R^2$ für gegebenenfalls einfach bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

Ganz besonders bevorzugt sind Verbindungen der Formel (I), bei welchen

$R^1$ für Wasserstoff, Methyl, Ethyl, n- oder i-Propyl, n-, i-, s- oder t-Butyl, Methoxy, Ethoxy, Methylthio oder Ethylthio steht und

$R^2$ für gegebenenfalls einfach oder zweifach, gleich oder verschieden substituiertes Phenyl steht, wobei als Substituenten infrage kommen:
Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio.

Im einzelnen seien außer den bei den Herstellungsbeispielen genannten Verbindungen die folgenden substituierten Pyridyltriazine der allgemeinen Formel (I) genannt:

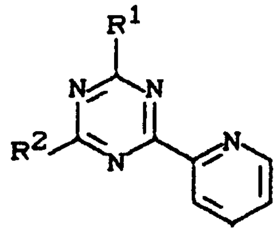

(I)

| $R^1$ | $R^2$ | $R^1$ | $R^2$ |
|---|---|---|---|
| $t-C_4H_9$ | 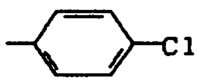 | H | 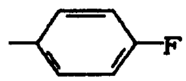 |
| $t-C_4H_9$ | 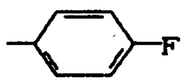 | H | 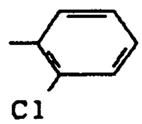 |
| $n-C_4H_9$ | 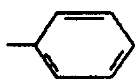 | H | 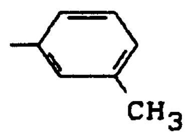 |
| $i-C_3H_7$ | 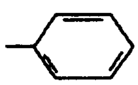 | $-S-CH_3$ | 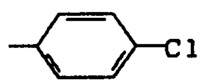 |
| $i-C_3H_7$ | 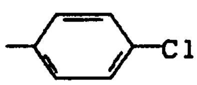 | $-S-CH_3$ | 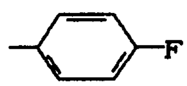 |
| $n-C_3H_7$ | 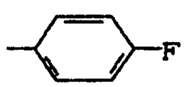 | $C_2H_5$ | 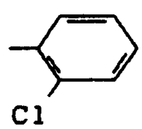 |
| $C_2H_5$ | 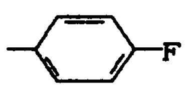 | $C_2H_5$ | 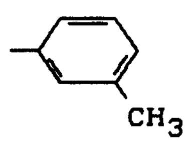 |
| $C_2H_5$ | 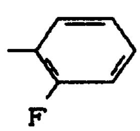 | | |

| $R^1$ | $R^2$ | $R^1$ | $R^2$ |
|---|---|---|---|
| $CH_3$ | 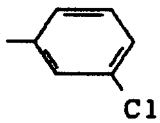 | $C_2H_5$ | 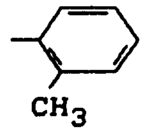 |
| H | 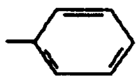 | $C_2H_5$ | 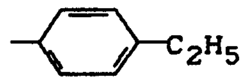 |
| H | 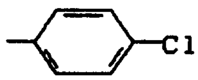 | $C_2H_5$ | 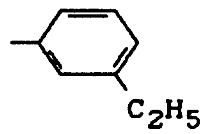 |
| $CH_3$ | 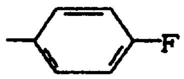 | $-S-C_2H_5$ | 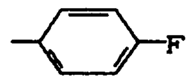 |
| $CH_3$ | 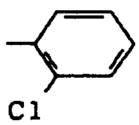 | $-S-C_2H_5$ | 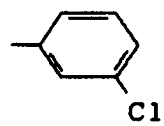 |
| $-O-C_2H_5$ | 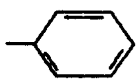 | $-O-CH_3$ | 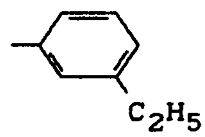 |
| $-S-CH_3$ | 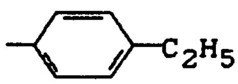 | $-O-CH_3$ | 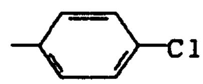 |
| $-S-CH_3$ | 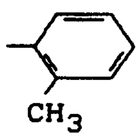 | $-O-C_2H_5$ | 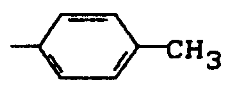 |
| $-S-C_2H_5$ | 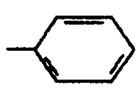 | | |

Verwendet man beispielsweise 2-Pyridylamidinhydrochlorid und N-Acetyl-benzolimidocarbonsäuremethylester als Ausgangsverbindungen, so läßt sich der Reaktionsablauf des erfindungsgemäßen Verfahren (a) durch das folgende Formelschema darstellen:

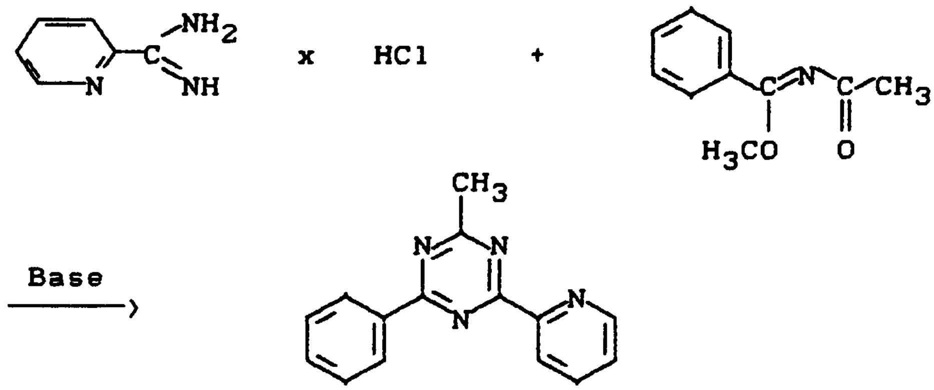


Verwendet man beispielsweise 2-Mercapto-4-(4-chlorphenyl)-6-(2-pyridyl)-1,3,5-triazin und Raney-Nickel als Ausgangsverbindungen, so läßt sich der Reaktionsablauf des erfindungsgemäßen Verfahren (b) durch das folgende Formelschema darstellen:

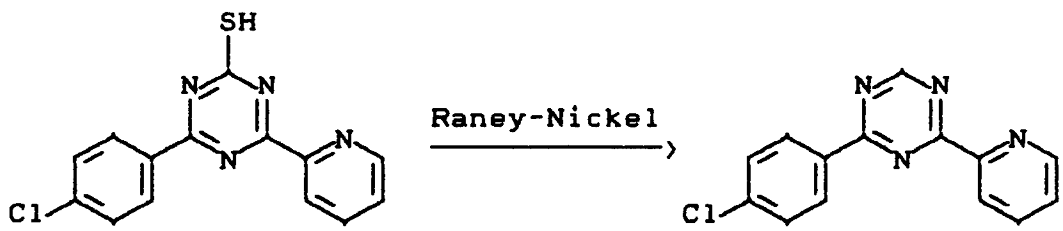


Verwendet man beispielsweise 2-Mercapto-4-(4-methylphenyl)-6-(2-pyridyl)-1,3,5-triazin und Methyliodid als Ausgangsverbindungen, so läßt sich der Reaktionsablauf des erfindungsgemäßen Verfahren (c) durch das folgende Formelschema darstellen:

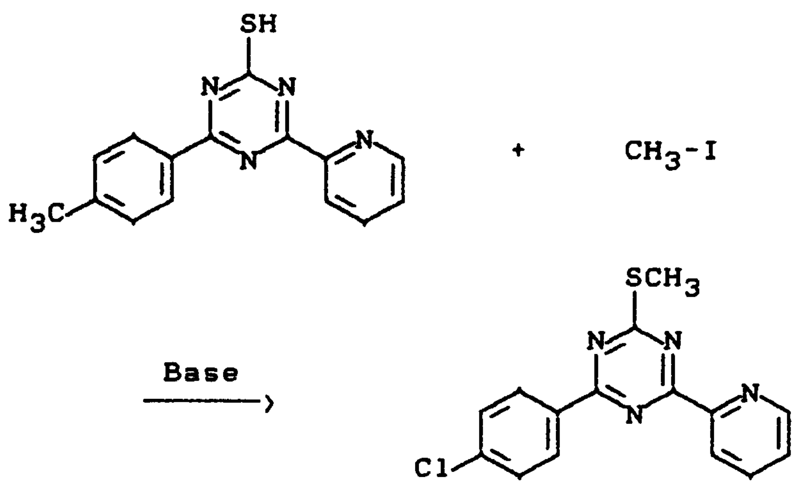


Verwendet man beispielsweise 2-Methylthio-4-(2-fluorphenyl)-6-(2-pyridyl)-1,3,5-triazin und Methanol als Ausgangsverbindungen, so läßt sich der Reaktionsablauf des erfindungsgemäßen Verfahren (d) durch das folgende Formelschema darstellen:

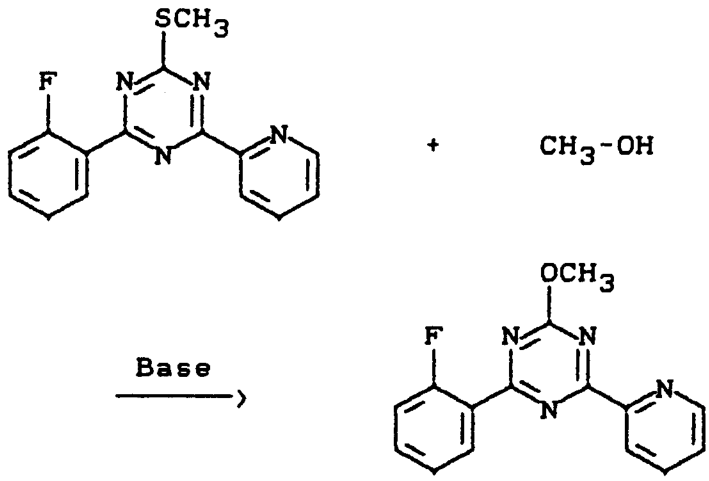


Das zur Durchführung des erfindungsgemäßen Verfahrens (a) als Ausgangsverbindung benötigte Pyridylamidin- bwz. dessen Säureadditionssalze sind durch die Formel (II) definiert. In dieser Formel (II) steht X vorzugsweise für das Anion einer anorganischen Mineralsäure, insbesondere für ein Halogenanion, wie Chlorid, Bromid oder Jodid oder für ein Hydrogensulfat- oder Hydrogencarbonatanion. Das Pyridylamidin bzw. dessen Säureadditionssalze der Formel (II) sind bekannt oder erhältlich im Analogie zu bekannten Verfahren (vgl. z.B. EP 259 139 oder EP 270 362).

Die zur Durchführung des erfindungsgemäßen Verfahrens (a) weiterhin als Ausgangsstoffe benötigten Imidsäureester sind durch die Formel (III) allgemein definiert. In dieser Formel (III) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden. $R^{1-1}$ steht vorzugsweise für geradkettiges oder verzweigtes Alkyl mit 1 bis 6, insbesondere mit 1 bis 4 Kohlenstoffatomen. $R^5$ steht vorzugsweise für geradkettiges oder verzweigtes Alkyl mit 1 bis 4 Kohlenstoffatomen oder für Phenyl. Die Imidsäureester der Formel (III) sind bekannt oder erhältlich in Analogie zu bekannten Verfahren (vgl. z.B. Synthesis 1983, 483).

Die zur Durchführung der erfindungsgemäßen Verfahren (b) und (c) als Ausgangsstoffe benötigten Mercapto-triazinylpyridine sind durch die Formel (IV) allgemein definiert. In dieser Formel (IV) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden. Die Mercaptotriazinylpyridine der Formel (IV) sind noch nicht bekannt und ebenfalls Gegenstand der Erfindung. Man erhält sie entweder, wenn man 2-Pyridylimidsäureester der Formel (VII)

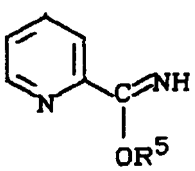 (VII)

in welcher

$R^5$ für Alkyl oder Aryl steht,

zunächst in einer ersten Stufe mit Benzoylchloriden der Formel (VIII),

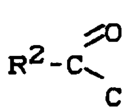 (VIII)

in welcher

$R^2$ die oben angegebene Bedeutung hat,

gegebenenfalls in Gegenwart eines Verdünnungsmittels wie beispielsweise Toluol und gegebenenfalls in

Gegenwart eines Reaktionshilfsmittels wie beispielsweise Triethylamin bei Temperaturen zwischen -20°C und +60°C umsetzt und anschließend die so erhältlichen N-acylierten 2-Pyridyl-imidsäureester der Formel (IX),

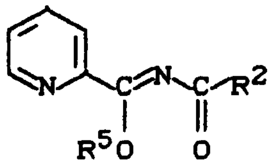

(IX)

$R^2$ und $R^5$ die oben angegebene Bedeutung haben,
mit Thioharnstoff gegebenenfalls in Gegenwart eines Verdünnungsmittels wie beispielsweise Methanol umsetzt oder wenn man Pyridylamidin oder dessen Säureadditionssalze der Formel (II)

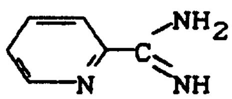

x HX (II)

in welcher
X die oben angegebene Bedeutung hat,
mit Benzoyl-isothiocyanaten der Formel (X),

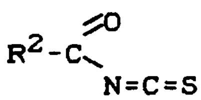

(X)

in welcher
$R^2$ die oben angegebene Bedeutung hat,
gegebenenfalls in Gegenwart eines Verdünnungsmittels wie beispielsweise einer Mischung aus Wasser und Toluol und gegebenenfalls in Gegenwart eines Reaktionshilfsmittels wie beispielsweise Natriumhydroxid bei Temperaturen zwischen -20°C und +60°C umsetzt.

Die so erhältlichen Mercapto-triazinylpyridine der Formel (IV) können direkt in einem sogenannten "Eintopfverfahren" gemäß den erfindungsgemäßen Verfahren (b) oder (c) weiter umgesetzt werden,

Die 2-Pyridylimidsäureester der Formel (VII) sind bekannt oder erhältlich in Analogie zu bekannten Verfahren (vgl. z.B. Chem. Letters 1975, 67-70). Benzoylchloride der Formel (VIII) und Benzoyl-isothiocyanate der Formel (X) sind allgemein bekannte Verbindungen der organischen Chemie oder erhältlich in Analogie zu allgemein bekannten Verfahren,

Die zur Durchführung des erfindungsgemäßen Verfahrens (c) weiterhin als Ausgangsstoffe benötigten Alkylierungsmittel sind durch die Formel (V) allgemein definiert. In dieser Formel (V) steht $R^3$ vorzugsweise für geradkettiges oder verzweigtes Alkyl mit 1 bis 6, insbesondere 1 bis 4 Kohlenstoffatomen. E steht für einen bei Alkylierungsmitteln üblichen Abgangsrest, vorzugsweise für Halogen, insbesondere Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy. Die Alkylierungsmittel der Formel (V) sind allgemein bekannte Verbindungen der organischen Chemie.

Die zur Durchführung des erfindungsgemäßen Verfahrens (d) als Ausgangsstoffe benötigten Alkylthiotriazinylpyridine sind durch die Formel (Ia) allgemein definiert. In dieser Formel (Ia) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden, $R^3$ steht vorzugsweise für geradkettiges oder verzweigtes Alkyl mit 1 bis 6, insbesondere 1 bis 4 Kohlenstoffatomen.

Die Alkylthio-triazinylpyridine der Formel (Ia) sind erfindungsgemäße Verbindungen und erhältlich mit Hilfe des erfindungsgemäßen Verfahrens (c).

Die zur Durchführung des erfindungsgemäßen Verfahrens (d) als Ausgangsstoffe benötigten Alkohole sind durch die Formel (VI) allgemein definiert. In dieser Formel (VI) steht $R^4$ vorzugsweise für geradkettiges

oder verzweigtes Alkyl mit 1 bis 6, insbesondere 1 bis 4 Kohlenstoffatomen,
Die Alkohole der Formel (VI) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol oder deren Gemische mit Wasser sowie reines Wasser.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden, Im allgemeinen arbeitet man bei Temperaturen zwischen 20 °C und 140 °C, vorzugsweise bei Temperaturen zwischen 60 °C und 100 °C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol an Pyridylamidin oder eines entsprechenden Säureadditionssalzes der Formel (II) allgemeinen 1.0 bis 3,0 Mol, vorzugsweise 1.0 bis 1,5 Mol an Imidsäureester der Formel (III) und gegebenenfalls 1.0 bis 5,0 Mol, vorzugsweise 1,0 bis 2,5 Mol an als Reaktionshilfsmittel verwendeter Base ein, Die Reaktionsdurchführung, Aufarbeitung und Isolierung der Reaktionsprodukte erfolgt nach allgemein üblichen Methoden (vgl. hierzu auch die Herstellungsbeispiele).

Als Verdünnungsmittel zur Durchführung des erfindungsgemäßen Verfahrens (b) kommen polare, mit Wasser mischbare organische Lösungsmittel infrage. Hierzu gehören insbesondere Ether, wie Dioxan, Tetrahydrofuran oder Ethylenglykoldimethyl- oder -diethylether oder Alkohole, wie Methanol, Ethanol, n- oder i-Propanol, deren Gemische mit Wasser oder reines Wasser.

Das erfindungsgemäße Verfahren (b) kann gegebenenfalls in Gegenwart eines geeigneten Reaktionshilfsmittels durchgeführt werden. Als solches kommt insbesondere wäßriger Ammoniak infrage.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 40 °C und 100 °C.

Das erfindungsgemäße Verfahren (b) wird üblicherweise unter Normaldruck durchgeführt. Es ist jedoch auch möglich unter erhöhtem Druck zu arbeiten.

Zur Durchführung des erfindungsgemäßen Verfahrens (b) setzt man pro Mol an Mercapto-triazinylpyridin der Formel (IV) im allgemeinen 1.0 bis 5,0 Mol, vorzugsweise 1.0 bis 2,5 Mol an Raney-Nickel und gegebenenfalls 1.0 bis 5,0 Mol, vorzugsweise 1,0 bis 2,5 Mol an Reaktionshilfsmittel ein.
In einer besonders bevorzugten Durchführungsvariante stellt man das als Ausgangsverbindung verwendete Mercapto-triazinylpyridin der Formel (IV) in einer vorgelagerten Reaktion direkt im Reaktionsgefäß her und setzt die so erhältliche Reaktionsmischung ohne Isolierung des Mercapto-triazinylpyridin der Formel (IV) in einer sogenannten "Eintopfreaktion" direkt anschließend gemäß dem erfindungsgemäßen Verfahren (b) mit Raney Nickel weiter um. Die Reaktionsdurchführung, Aufarbeitung und Isolierung der Reaktionsprodukte erfolgt nach bekannten Verfahren (vgl. hierzu auch die Herstellungsbeispiele).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyl- oder -diethylether; Ketone wie Aceton oder Butanon oder Methyl-isobutyl-keton; Nitrile wie Acetonitril, Propionitril oder Benzonitril; Amide wie N,N-Dimethylformamid, N,N-Dimethylacetamid, N-Methylformanilid, N-Methylpyrrolidon oder Hexamethylphosphorsäuretriamid, Ester wie Essigsäuremethylester oder Essigsäureethylester oder Sulfoxide wie Dimethylsulfoxid, Alkohole, wie Methanol, Ethanol, n- oder i-Propanol oder deren

Gemische mit Wasser sowie reines Wasser.

Das erfindungsgemäße Verfahren (c) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}$/$C_{15}$-alkylammoniumchlorid, Trimethyl-$C_{13}$/$C_{15}$-alkylammoniumbromid, Dibenzyl-dimethylammoniummethylsulfat, Dimethyl-$C_{12}$/$C_{14}$-alkyl-benzylammoniumchlorid, Dimethyl-$C_{12}$/$C_{14}$-alkyl-benzylammoniumbromid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (c) wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +80 °C, vorzugsweise bei Temperaturen zwischen 0 °C und +60 °C.

Zur Durchführung des erfindungsgemäßen Verfahrens (c) setzt man pro Mol an Mercapto-triazinylpyridin der Formel (IV) im allgemeinen 1.0 bis 3,0 Mol, vorzugsweise 1.0 bis 1,5 Mol an Alkylierungsmittel der Formel (V) und gegebenenfalls 1.0 bis 5,0 Mol, vorzugsweise 1.0 bis 2,5 Mol an als Reaktionshilfsmittel verwendeter Base ein.

In einer besonders bevorzugten Durchführungsvariante stellt man das als Ausgangsverbindung verwendete Mercapto-triazinylpyridin der Formel (IV) in einer vorgelagerten Reaktion direkt im Reaktionsgefäß her und setzt die so erhältiche Reaktionsmischung ohne Isolierung des Mercapto-triazinylpyridin der Formel (IV) in einer sogenannten "Eintopfreaktion" direkt anschließend gemäß dem erfindungsgemäßen Verfahren (c) mit dem Alkylierungsmittel der Formel (V) weiter um. Die Reaktionsdurchführung, Aufarbeitung und Isolierung der Reaktionsprodukte erfolgt nach bekannten Verfahren (vgl. hierzu auch die Herstellungsbeispiele).

Als Verdünnungsmittel zur Durchführung des erfindungsgemäßen Verfahrens (d) kommen polare, mit Wasser mischbare organische Lösungsmittel infrage. Hierzu gehören insbesondere Ether, wie Dioxan, Tetrahydrofuran oder Ethylenglykoldimethyl- oder -diethylether oder Alkohole, wie Methanol, Ethanol, n- oder i-Propanol, deren Gemische mit Wasser oder reines Wasser.

Mit besonderem Vorzug verwendet man einen entsprechenden Überschuß an dem als Reaktionspartner verwendeten Alkohol der Formel (VI) gleichzeitig als Verdünnungsmittel.

Das erfindungsgemäße Verfahren (d) wird vorzugsweise in Gegenwart eines geeigneten Reaktionshilfsmittels durchgeführt. Als solche kommen alle üblicherweise verwendbaren anorganischen und organischen Basen infrage. Mit besonderem Vorzug verwendet man ein dem als Reaktionspartner verwendeten Alkohol der Formel (VI) entsprechendes Alkalimetallalkoholat als Reaktionshilfsmittel.

Die Reaktionstemperaturen können bei der Durchführung des erfindungsgemäßen Verfahrens (d) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (d) setzt man pro Mol an Alkylthio-triazinylpyridin der Formel (Ia) im allgemeinen 1.0 bis 20,0 Mol, vorzugsweise 1.0 bis 10,0 Mol an Alkohol der Formel (VI) und gegebenenfalls 1.0 bis 2,0 Mol, vorzugsweise 1.0 bis 1,2 Mol an als Reaktionshilfsmittel verwendeter Base ein. Die Reaktionsdurchführung, Aufarbeitung und Isolierung der Reaktionsprodukte erfolgt nach allgemein üblichen Verfahren (vgl. hierzu auch die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:

Pythium-Arten, wie beispielsweise Pythium ultimum; Phytophthora-Arten, wie beispielsweise Phytophthora infestans; Pseudoperonospora-Arten, wie beispielsweise Pseudoperonospora humuli oder Pseudoperonospora cubensis; Plasmopara-Arten, wie beispielsweise Plasmopara viticola; Peronospora-Arten, wie beispielsweise Peronospora pisi oder Peronospora brassicae; Erysiphe-Arten, wie beispielsweise Erysiphe

graminis; Sphaerotheca-Arten, wie beispielsweise Sphaerotheca fuliginea; Podosphaera-Arten, wie beispielsweise Podosphaera leucotricha; Venturia-Arten, wie beispielsweise Venturia inaequalis; Pyrenophora-Arten, wie beispielsweise Pyrenophora teres oder Pyrenophora graminea (Konidienform: Drechslera, Synonym: Helminthosporium); Cochliobolus-Arten, wie beispielsweise Cochliobolus sativus (Konidienform: Drechslera, Synonym: Helminthosporium); Uromyces-Arten, wie beispielsweise Uromyces appendiculatus; Puccinia-Arten, wie beispielsweise Puccinia recondita; Tilletia-Arten, wie beispielsweise Tilletia caries; Ustilago-Arten, wie beispielsweise Ustilago nuda oder Ustilago avenae; Pellicularia-Arten, wie beispielsweise Pellicularia sasakii; Pyricularia-Arten, wie beispielsweise Pyricularia oryzae; Fusarium-Arten, wie beispielsweise Fusarium culmorum; Botrytis-Arten, wie beispielsweise Botrytis cinerea; Septoria-Arten, wie beispielsweise Septoria nodorum; Leptosphaeria-Arten, wie beispielsweise Leptosphaeria nodorum; Cercospora-Arten, wie beispielsweise Cercospora canescens; Alternaria-Arten, wie beispielsweise Alternaria brassicae; Pseudocercosporella-Arten, wie beispielsweise Pseudocercosporella herpotrichoides. Die gute Pflanzenverträglichkeit der Wirkstoffe in den zur Bekämpfung von Pflanzenkrankheiten notwendigen Konzentrationen erlaubt eine Behandlung von oberiridischen Pflanzenteilen, von Pflanz- und Saatgut und des Bodens.

Dabei können die erfindungsgemäßen Wirkstoffe mit besonders gutem Erfolg zur Bekämpfung von Getreidekrankheiten, wie beispielsweise gegen den Erreger der Braunfleckenkrankheit an Gerste (Cochliobolus sativus) oder gegen den Erreger der Braunspelzigkeit des Weizens (Leptosphaeria nodorum) oder gegen den Erreger des echten Getreidemehltaues (Erysiphe graminis) oder zur Bekämpfung von Krankheiten im Obst- und Gemüseanbau, wie beispielsweise gegen den Erreger des Apfelschorfes (Venturia inaequalis) oder gegen den Erreger des Rebenmehltaues (Uncinula necator) oder zur Bekämpfung von Reiskrankheiten, wie beispielsweise gegen den Erreger der Reisfleckenkrankheit (Pyricularia oryzae) eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Parafine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Herstellungsbeispiele:

Beispiel 1:

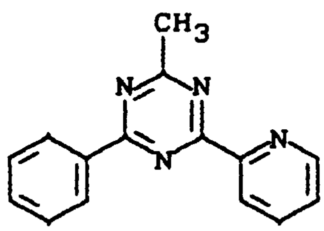

(Verfahren (a))

5,2 g (0,033 Mol) Pyridin-2-carbamidin-hydrochlorid (vgl. z.B. EP 259 139 oder EP 270 362), 5,8 g (0,033 Mol) N-Acetylbenzimidsäuremethylester und 8,4 g (0,039 Mol) einer 20%igen Natriummethylat-Lösung in Methanol werden in 60 ml Methanol für 3 Tage auf Rückflußtemperatur erhitzt, anschließend auf Raumtemperatur abgekühlt, im Vakuum eingeengt, der Rückstand in Dichlormethan aufgenommen, mit Wasser gewaschen, über Natriumsulfat getrocknet, abermals im Vakuum eingeengt und der Rückstand über Kieselgel chromatographiert (Laufmittel: n-Hexan/Aceton 7:3).

Man erhält 2,1 g (26% der Theorie) an 2-(2-Pyridyl)-4-phenyl-6-methyl-1,3,5-triazin vom Schmelzpunkt 97 °C.

Beispiel 2:

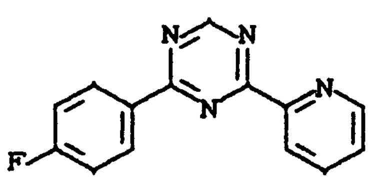

(Verfahren (b))

Zu einer Lösung von 5,6 g (0,02 Mol) 2-(2-Pyridyl)-4-(4-fluorphenyl)-6-mercapto-1,3,5-triazin in einer Mischung aus 200 ml Ethanol, 200 ml Wasser und 50 ml konzentriertem wäßrigem Ammoniak gibt man 5 g

feuchtes Raney-Nickel und erhitzt für 6 Stunden auf Rückflußtemperatur. Zur Aufarbeitung wird die Lösung filtriert, das Filtrat auf ein Volumen von ca. 100 ml eingeengt, ausgefallener Niederschlag abgesaugt, in Methanol aufgenommen und filtriert. Das Filtrat wird eingeengt, der Rückstand in Essigsäureethylester aufgenommen, mit Wasser gewaschen, über Natriumsulfat getrocknet und eingeengt. Der ölige Rückstand wird durch Verreiben mit Ether/Petrolether zur Kristallisation gebracht.

Man erhält 0,5 g (10% der Theorie) an 2-(2-Pyridyl)-4-(4-fluorphenyl)-1,3,5-triazin vom Schmelzpunkt Fp. > 180 °C (Zers.).

Herstellung der Ausgangsverbindung:

Beispiel IV-1:

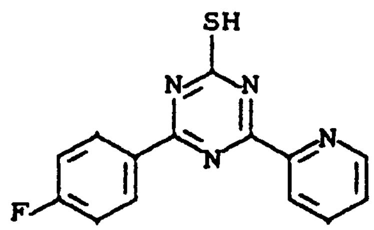

3,2 g (0,02 Mol) Pyridin-2-carbamidin-hydrochlorid werden in 40 ml Wasser gelöst und mit 80 ml Toluol überschichtet. Unter starkem Rühren gibt man gleichzeitig jeweils tropfenweise innerhalb von 20 Minuten 20 ml 4N-Natronlauge und eine Lösung von 3,6 g (0,02 Mol) 4-Fluorbenzoylisothiocyanat in 20 ml Toluol zu und rührt anschließend weitere 18 Stunden bei 20 °C. Zur Aufarbeitung wird soviel Methanol zugegeben, daß eine klare, zweiphasige Lösung entsteht. Die wäßrige Phase wird abgetrennt, mit 2N-Schwefelsäure auf pH 5 gebracht, ausgefallener Niederschlag abgesaugt und getrocknet.

Man erhält 4,0 g (70% der Theorie) an 2-(2-Pyridyl)-4-(4-fluorphenyl)-6-mercapto-1,3,5-triazin vom Schmelzpunkt >270 °C.

Beispiel 3:

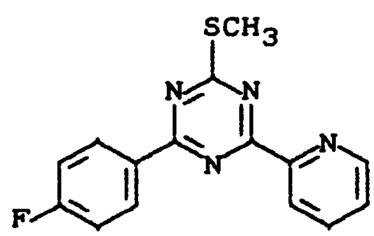

(Verfahren (c))

Zu 1,7 g (0,006 Mol) 2-(2-Pyridyl)-4-(4-fluorphenyl)-6-mercapto-1,3,5-triazin und 18 ml 0,5 N-Natronlauge in 40 ml Ethanol gibt man 0,9 g (0,0066 Mol) Methyliodid, rührt anschließend 30 Minuten bei Raumtemperatur, saugt dann den entstehenden Niederschlag ab und trocknet ihn.

Man erhält 1,5 g (84% der Theorie) an 2-(2-Pyridyl)-4-(4-fluorphenyl)-6-methylthio-1,3,5-triazin vom Schmelzpunkt 158 °C.

Beispiel 4:

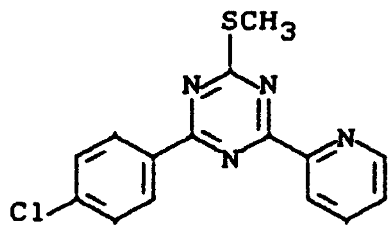

(Verfahren (c) - Eintopfverfahren)

Zu einer Lösung von 0,6 g (0,011 Mol) Natriummethylat und 0,8 g (0,01 Mol) Thioharnstoff in 50 ml Methanol gibt man 2,7 g (0,01 Mol) N-(4-Chlorbenzoyl)-pyridin-2-imidocarbonsäuremethylester, erhitzt anschließend für 3 Stunden auf Rückflußtemperatur, gibt dann 1,8 g (0,0125 Mol) Methyliodid zu und rührt weitere 18 Stunden bei Raumtemperatur. Der ausgefallene Niederschlag wird abgesaugt und getrocknet.

Man erhält 0,4 g (13% der Theorie) an 2-(2-Pyridyl)-4-(4-chlorphenyl)-6-methylthio-1,3,5-triazin vom Schmelzpunkt 152 ° C.

Beispiel 5:

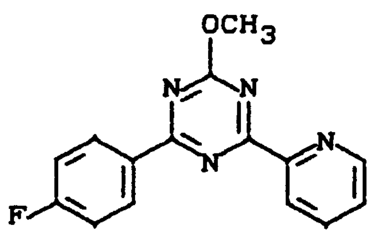

(Verfahren (d))

Zu einer Lösung von 0,5 g (0,01 Mol) Natriummethylat in 100 ml Methanol gibt man 3,0 g (0,01 Mol) 2-(2-Pyridyl)-4-(4-fluorphenyl)-6-methylthio-1,3,5-triazin und erhitzt anschließend für 6 Stunden auf Rückflußtemperatur. Zur Aufarbeitung wird die Reaktionsmischung auf ca. 20 ml eingeengt, abgekühlt und der ausgefallene Niederschlag wird abgesaugt und getrocknet.

Man erhält 2,6 g (92% der Theorie) an 2-(2-Pyridyl)-4-(4-fluorphenyl)-6-methoxy-1,3,5-triazin vom Schmelzpunkt >270 ° C.

In entsprechender Weise und gemäß den allgemeinen Angaben zur Herstellung erhält man die folgenden substituierten Pyridyltriazine der allgemeinen Formel (I),

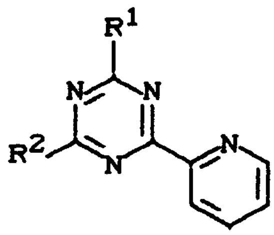

(I)

| Bsp.-Nr. | $R^1$ | $R^2$ | Schmelzpunkt / °C |
|---|---|---|---|
| 6 | $t\text{-}C_4H_9$ | 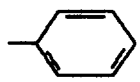 | 71 |
| 7 | $CH_3$ | 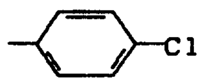 | 121 |
| 8 | $C_2H_5$ | 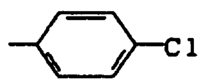 | 112 |
| 9 | $C_2H_5$ | 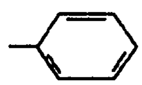 | 80 |
| 10 | $n\text{-}C_3H_7$ | 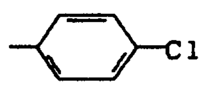 | 65 |
| 11 | $n\text{-}C_3H_7$ | 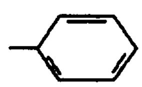 | 102 |
| 12 | $-SCH_3$ | 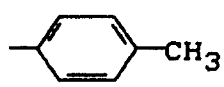 | 165 |
| 13 | $-SCH_3$ | 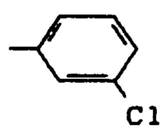 | 243 |
| 14 | $-OCH_3$ | 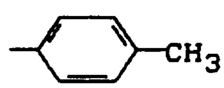 | >275 |

Anwendungsbeispiele:

In den folgenden Anwendungsbeispielen wurden die nachstehend aufgeführten Verbindungen als Vergleichssubstanzen eingesetzt:

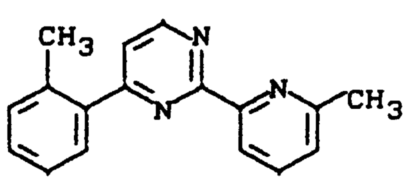

(A)

2-(6-Methyl-2-pyridyl)-4-(2-methylphenyl)-pyrimidin

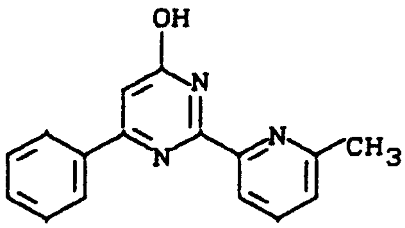

(B)

2-(6-Methyl-2-pyridyl)-4-phenyl-6-hydroxy-pyrimidin

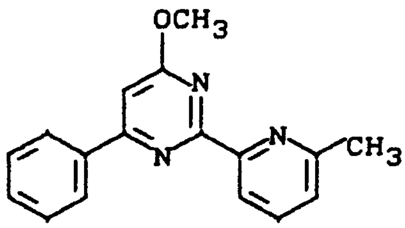

(C)

2-(6-Methyl-2-pyridyl)-4-phenyl-6-methoxy-pyrimidin
(alle bekannt aus EP 270 362)

Beispiel A:

Venturia-Test (Apfel)/protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1, 8 und 9.

Beispiel B

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 8 und 9.

Beispiel C:

Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel: 10.

Beispiel D:

Uncinula-Test (Rebe)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden anschließend im Gewächshaus bei 23 °C bis 24 °C und einer relativen Luftfeuchtigkeit von ca. 75% aufgestellt.

14 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 4, 7, 8, 10 und 11.

**Patentansprüche**

1. Substituierte Pyridyltriazine der allgemeinen Formel (I)

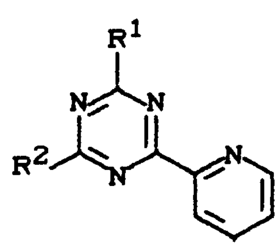

(I)

in welcher

$R^1$ für Wasserstoff, Alkyl, Alkoxy oder Alkylthio steht und

$R^2$ für gegebenenfalls substituiertes Phenyl steht.

**2.** Substituierte Pyridyltriazine der Formel (I) gemäß Anspruch 1, bei welchen
$R^1$ für Wasserstoff, für geradkettiges oder verzweigtes Alkyl mit 1 bis 6 Kohlenstoffatomen, für geradkettiges oder verzweigtes Alkoxy mit 1 bis 6 Kohlenstoffatomen oder für geradkettiges oder verzweigtes Alkylthio mit 1 bis 6 Kohlenstoffatomen steht und
$R^2$ für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen oder gegebenenfalls einfach oder mehrfach, gleich oder verschieden durch Halogen und/oder geradkettiges oder verzweigtes Alkyl mit 1 bis 4 Kohlenstoffatomen substituiertes Phenyl.

**3.** Substituierte Pyridyltriazine der Formel (I) gemäß Anspruch 1, bei welchen
$R^1$ für Wasserstoff, für geradkettiges oder verzweigtes Alkyl mit 1 bis 4 Kohlenstoffatomen, für geradkettiges oder verzweigtes Alkoxy mit 1 bis 4 Kohlenstoffatomen oder für geradkettiges oder verzweigtes Alkylthio mit 1 bis 4 Kohlenstoffatomen steht und
$R^2$ für gegebenenfalls einfach bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl.

**4.** Substituierte Pyridyltriazine der Formel (I) gemäß Anspruch 1, bei welchen
$R^1$ für Wasserstoff, Methyl, Ethyl, n- oder i-Propyl, n-, i-, s- oder t-Butyl, Methoxy, Ethoxy, Methylthio oder Ethylthio steht und
$R^2$ für gegebenenfalls einfach oder zweifach, gleich oder verschieden substituiertes Phenyl steht, wobei als Substituenten infrage kommen:
Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio.

**5.** Verfahren zur Herstellung von substituierten Pyridyltriazinen der Formel (I)

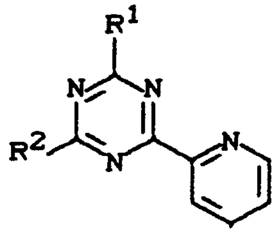

(I)

in welcher
$R^1$ für Wasserstoff, Alkyl, Alkoxy oder Alkylthio steht und
$R^2$ für gegebenenfalls substituiertes Phenyl steht,
dadurch gekennzeichnet, daß man
a) für den Fall, daß $R^1$ für Alkyl steht, Pyridylamidin oder dessen Säureadditionssalzen der Formel (II)

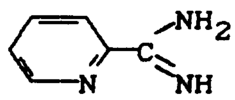 x HX (II)

in welcher
X für ein Anion einer anorganischen Säure steht,
mit Imidsäureestern der Formel (III),

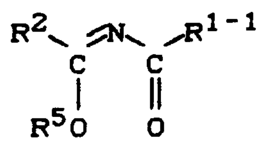 (III)

in welcher
$R^{1-1}$ für Alkyl steht,
$R^2$ die oben angegebene Bedeutung hat und
$R^5$ für Alkyl oder Aryl steht,
gegebenenfalls in Gegenwart eines Verdünnungsmittels und gegebenenfalls in Gegenwart eines Reaktionshilfsmittels umsetzt oder wenn man
b) für den Fall, daß $R^1$ für Wasserstoff steht, Mercapto-triazinylpyridine der Formel (IV),

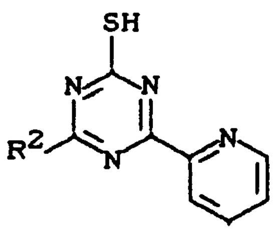 (IV)

in welcher
$R^2$ die oben angegebene Bedeutung hat,
mit Raney Nickel gegebenenfalls in Gegenwart eines Verdünnungsmittels entschwefelt oder
c) für den Fall, daß $R^1$ für Alkylthio steht, die Verbindungen der Formel (IV) mit Alkylierungsmitteln der Formel (V),

$R^3$-E (V)

in welcher
$R^3$ für Alkyl steht und
E für eine elektronenanziehende Abgangsgruppe steht,
gegebenenfalls in Gegenwart eines Verdünnungsmittels und gegebenenfalls in Gegenwart eines Reaktionshilfsmittels umsetzt oder wenn man
d) für den Fall, daß $R^1$ für Alkoxy steht, die mit Hilfe des erfindungsgemäßen Verfahrens (c) erhältlichen Alkylthio-triazinylpyridine der Formel (Ia),

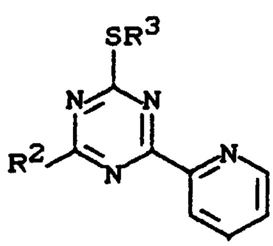 (Ia)

in welcher
$R^3$ für Alkyl steht und
$R^2$ die oben angegebene Bedeutung hat,
mit Alkoholen der Formel (VI),

$R^4$-OH (VI)

in welcher
$R^4$ für Alkyl steht,
gegebenenfalls in Gegenwart eines Verdünnungsmittels und gegebenenfalls in Gegenwart eines Reaktionshilfsmittels umsetzt.

**6.** Schädlingsbekämpfungsmittel, gekennzeichnet durch einen Gehalt an mindestens einem substituierten Pyridyltriazin der Formel (I) nach den Ansprüchen 1 und 5.

**7.** Verwendung von substituierten Pyridyltriazinen der Formel (I) nach den Ansprüchen 1 und 5 zur Bekämpfung von Schädlingen.

**8.** Verfahren zur Bekämpfung von Schädlingen, dadurch gekennzeichnet, daß man substituierte Pyridyltriazine der Formel (I) nach den Ansprüchen 1 und 5 auf Schädlinge und/oder ihren Lebensraum einwirken läßt.

**9.** Verfahren zur Herstellung von Schädlingsbekämpfungsmitteln, dadurch gekennzeichnet, daß man substituierte Pyridyltriazine der Formel (I) nach den Ansprüchen 1 und 5 mit Streckmitteln und/oder oberflächenaktiven Mitteln vermischt.

**10.** Mercaptosubstituierte Pyridyltriazin der Formel (IV)

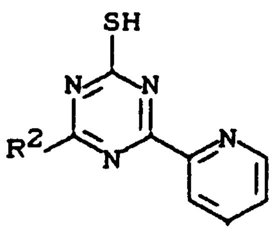

(IV)

in welcher
$R^2$ für gegebenenfalls substituiertes Phenyl steht.

**11.** Verfahren zur Herstellung von mercaptosubstituierten Pyridyltriazine der Formel (IV)

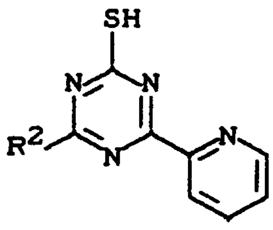

(IV)

in welcher
$R^2$ für gegebenenfalls substituiertes Phenyl steht, dadurch gekennzeichnet, daß man 2-Pyridylimidsäureester der Formel (VII)

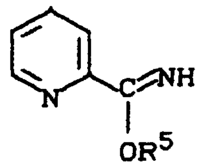

(VII)

in welcher
$R^5$ für Alkyl oder Aryl steht,
zunächst in einer ersten Stufe mit Benzoylchloriden der Formel (VIII),

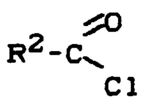

(VIII)

in welcher
$R^2$ die oben angegebene Bedeutung hat,
gegebenenfalls in Gegenwart eines Verdünnungsmittels und gegebenenfalls in Gegenwart eines Reaktionshilfsmittels bei Temperaturen zwischen -20°C und +60°C umsetzt und anschließend die so erhältlichen N-acylierten 2-Pyridyl-imidsäureester der Formel (IX),

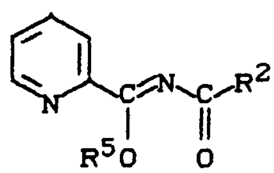

(IX)

in welcher
$R^2$ und $R^5$ die oben angegebene Bedeutung haben,
mit Thioharnstoff gegebenenfalls in Gegenwart eines Verdünnungsmittels umsetzt oder daß man Pyridylamidin oder dessen Säureadditionssalze der Formel (II)

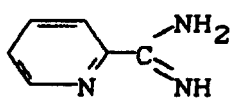

x HX (II)

in welcher
X für das Anion einer anorganischen Säure steht,
mit Benzoyl-isothiocyanaten der Formel (X),

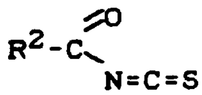

(X)

in welcher
$R^2$ die oben angegebene Bedeutung hat,
gegebenenfalls in Gegenwart eines Verdünnungsmittels und gegebenenfalls in Gegenwart eines Reaktionshilfsmittels bei Temperaturen zwischen -20°C und +60°C umsetzt.

Europäisches Patentamt

# EUROPÄISCHER RECHERCHENBERICHT

Nummer der Anmeldung

EP 92 11 4032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | WO-A-9 107 399 (HOEUCHST)<br>* Ansprüche *<br>--- | 1-3,5-7 | C07D401/04<br>A01N43/66 |
| A | TETRAHEDRON LETTERS<br>Bd. 22, Nr. 15, 1981, OXFORD GB<br>Seiten 1393 - 1396<br>H.FIGEYS, A.MATHY 'DIELS-ALDER REACTIONS WITH INVERSE ELECTRON DEMAND.'<br>* Seite 1393 - Seite 1395 *<br>----- | 1 | |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 26 NOVEMBER 1992 | FRANCOIS J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)